# EUROPEAN PATENT SPECIFICATION

(11) **EP 1 686 368 B1**
(45) Date of publication and mention of the grant of the patent: **04.01.2017**
(21) Application number: 05256885.4
(22) Date of filing: 07.11.2005
(51) Int. Cl.: C12M 1/34, G01N 1/28, G01N 21/64, G01N 21/25

(54) **Animal cell confluence detection apparatus and method**
Vorrichtung und Verfahren zur Erkennung des Zusammenflusses von Tierzellen
Appareil et procédé de détection de la confluence de cellules animales

(30) Priority: 27.01.2005 US 50826
(43) Date of publication of application: 02.08.2006
(73) Proprietor: Molecular Devices LLC, Sunnyvale, CA 94089 (US)
(72) Inventor: Jiang, Yonggang, New Milton Hampshire BH25 5NN (GB); Board, Andrew, New Milton Hampshire BH25 5NN (GB)
(74) Representative: Hartley, Andrew Philip

(56) References cited:
- EP-A1- 0 625 569
- WO-A-01/69206
- WO-A-95/09640
- WO-A-99/39184
- WO-A1-01/48147
- WO-A1-01/77648
- WO-A1-89/08841
- WO-A2-01/27591
- WO-A2-03/036265
- WO-A2-03/067904
- WO-A2-03/077008
- WO-A2-2004/017374
- WO-A2-2004/046307
- DD-A1- 125 735
- US-A- 3 712 746
- US-A- 4 601 551
- US-A1- 2002 072 117
- US-A1- 2002 090 320
- US-A1- 2004 032 589
- US-A1- 2004 229 210
- US-A1- 2004 233 545
- US-A1- 2005 014 255
- JONES P ET AL: "INTEGRATION OF IMAGE ANALYSIS AND ROBOTICS INTO A FULLY AUTOMATED COLONY PICKING AND PLATE HANDLING SYSTEM", NUCLEIC ACIDS RESEARCH, INFORMATION RETRIEVAL LTD, GB, vol. 20, no. 17, 1 January 1992 (1992-01-01), pages 4599-4606, XP002190262, ISSN: 0305-1048

## Description

### BACKGROUND OF THE INVENTION

The invention relates to an apparatus for and method of detecting confluence in animal cells.

It is common practice to culture animal cells in 96 well plates. However, it is a well known property of such colonies that they display contact inhibition whereby cell division ceases once the cells have grown across the well to fill the available area and touch each other. The degree to which the cells have grown to fill the well or other biological sample container is referred to as confluence, and one speaks of a well, plate or dish being 70% confluent, 80% confluent and so forth. The term subconfluent is also used to refer to a plate in which the cell colony or other cell aggregate has not yet reached confluence. If a colony is grown to high or full confluence this may also damage the experiment. For example, some cells grown at high confluence may lose their adherent phenotype.

Since the cell growth rate is not generally predictable, and since different colonies grow at different rates, the standard practice is for an operator to examine the well plates daily, or at longer or shorter regular intervals, by viewing the plate directly or under a microscope. Based on this visual inspection, the operator makes a decision on whether the cells should be disrupted and re-plated into larger wells, such as a 24 well plate or a 6 well plate. Often, the colonies are re-plated several times into progressively larger wells, e.g. from 96 to 24 to 6 well plates. For example, it is typical that replating will be performed if the cultures approach confluence, for example 65-75% confluence, or a lower degree of confluence, for example 50%, if the cells would be adversely affected if they became confluent.

The manual visual inspection for confluence is highly time-consuming and to a certain extent also non-auditable and non-repeatable in that it relies on human judgement and experience. Typically, it might take an experienced operator an hour to inspect a batch of 10 well plates.

It would therefore be desirable to automate the confluence detection process, in particular in a robot with well plate handling and cell picking capability.

The article "Integration of image analysis and robotics into a fully automated colony picking and plate handling system" by Peter Jones et al., Nucleic Acids Research, Vol. 20, N°17, and document WO03067904 show devices for analysing cell colonies.

A known method for detecting confluence is electrically using an impedance measurement. For example, extracellular electrode arrays can be used for capacitance measurements of adherent cells growing in colonies. Although possibly automatable, impedance measurement is generally viewed with suspicion, because it is considered undesirable to apply voltages to the cells in case this interferes with the cells in some way. An example of this method is disclosed in De Blasio et al, Biotechniques 2004 April 36(4), pages 650ff "Combining optical and electrical impedance techniques for quantitative measurement of confluence in MDCK-I cell culture" [1].

Phase contrast microscopy is another well known technique which can be used to image cell boundaries and thus detect confluence. However, it would be difficult and expensive to integrate a phase contrast microscope into a suitable robot. In particular, the inherent wavelength dependence of phase contrast microscopy makes it difficult to automate when viewing wells of standard well plates bearing in mind that the colony will often be an adherent one, adhered to the base and lower side walls of the well, which is at a refractive index discontinuity created by the material of the well plate and the liquid or air filling the well.

Although an automated optical approach would be desirable to replicate the manual inspection, the colorless and low-contrast nature of the usual cell boundaries makes this challenging.

### SUMMARY OF THE INVENTION

According to a first aspect of the invention, there is provided a process according to claim 1.

The biological sample container may be a well plate or other type of container such as a Petri dish, omni tray, Q-tray etc.

By the simple solution of illuminating from below at an oblique angle, it has been found that many animal cell types can be imaged with sufficient contrast to allow cell identification and consequent cell area computation using image processing techniques, thereby allowing confluence to be determined of animal cells being cultured in well plates or other receptacles. This avoids the need for more complex optical imaging techniques, such as phase contrast microscopy, and in many cases avoids the need for fluorescently tagging the cells or staining the cells. Moreover, this simple solution is amenable to automation in a picking robot with minimum disruption to other design features of a picking robot, such as head design and positioning, and well plate feeding and stacking. The animal cells could be individual cells, colonies of cells, cell monolayers or other kinds of cell aggregates.

The oblique angle at which the optical source illuminates the object position is between 10 to 50 degrees, and preferably 20 to 40 degrees, to the horizontal, with angles of around 30 degrees (25 to 35 degrees) being optimal for the systems used to date. The angle refers to the optical axis of the illumination.

The process can be applied iteratively to scan across all the wells of a well plate. For example, the optical source and the detector can be iteratively realigned relative to the well plate so that images of a sequence of wells in the well plate are collected and processed, whereby the degree of confluence of the animal cells is determined in a plurality of wells across the well plate. This can be achieved by mounting the optical source and detector on a common platform and mounting the platform on an xy-positioning system which is driven to move the optical source and detector together from well to well. Alternatively, the optical source and detector can remain static, and the well plate can be moved. This can be achieved by providing a well plate mounting platen or other form of carrier on the bed of the apparatus which is coupled to its own xy-positioning system. In any given apparatus, either one or both of these two xy-positioning systems could be provided.

The optical source can conveniently comprise a plurality of directional light emitting units arranged to emit beams having optical axes lying on the surface of a common cone, the point of which is coincident with the object position. Most conveniently, the directional light emitting units are arranged in a ring.

In some embodiments, the optical source comprises a plurality of directional light emitting units arranged to emit beams having optical axes lying on the surface of at least two cones whose points are coincident with each other and the object position. According to this design alternative, most conveniently the directional light emitting units are arranged in multiple concentric rings. This can provide a greater illumination power when all rings are illuminated simultaneously. Perhaps more importantly, each ring has its own characteristic illumination angle which is a key determinant for contrast of the cell perimeters in the dark field image, so that the ring that provides the greatest contrast in the image can be used for the confluence determination.

The light emitting units are LEDs in the main embodiment described below, but in other embodiments could be superfluorescent LEDs, lasers, in particular semiconductor lasers, or lamp sources, such as a Xenon lamp. The LEDs used in the main embodiment are white LEDs, but other embodiments could use UV LEDs or single color LEDs, or groups of single color LEDs of different color to produce broader band emission, such as white light. Groups of LEDs or other sources of two different colors may also be a useful combination for optimizing contrast or other purposes.

The image is preferably collected from below the object position. This provides a very convenient design, since both the illumination and collection optics are then arranged below the well plate, leaving the entire half space above the well plate free for plate handling mechanisms, cell picking head movement and other activities. The mechanical design of the cell picking and confluence detection functions can then be done largely separately, greatly simplifying the automation.

The optical source is preferably formed such that an open light path exists downwardly from the object position, and the light is collected via this open light path. A detector, such as a CCD camera, can thus be positioned to collect light scattered downwardly from the object position, and the light can be collected by the detector via this open light path. Alternatively, the image can be captured from above rather than below, so that light scattered upwardly from the object position is collected. For example, a CCD camera or other detector can be housed above the main bed of the apparatus in the roof or suspended from a gantry.

The degree of confluence can be determined by an automated cell count which is translated into an area by multiplication of the cell count by an area representing an average area for the cell type being cultured. Alternatively, the degree of confluence is determined by processing the image to: establish cell boundaries, compute the area of each cell from the cell boundary, and sum the cell areas. Image processing software, or alternatively any mixture of software, firmware and hardware, can be used to perform the image processing.

The cell boundaries can be determined directly by contrast from the plasma membrane, or from the extent of the cytoplasm, or possibly in some cases from contrast provided by an extracellular fluid in which the cell is located. Although testing to date has indicated that no fluorescence staining is necessary in many cell types of interest, modifying the cells by inclusion of a fluorescent tag may be performed, e.g. to image other cell parts, such as the nucleus, or to assess the physiological state of the cell, such as cell cycle. For example, red lectin can be used to tag the cell membranes. Nuclear tags that do not kill the cells may also be suitable to provide contrast in the case that the aggregate area of the cells is determined by cell counting rather than by direct cell area calculation. Whole cell stains may also be considered, such as Phalloidin FM4-64. A variety of suitable dyes are known and can be selected, for example, from the Molecular Probes catalog.

According to a second aspect of the invention, there is provided an apparatus according to claim 10.

For full automation, a well plate feeder/stacker, or feeder/stacker for other type of biological sample container, is preferably provided to supply each of a plurality of well plates from a feed, typically a well plate storage cassette, to the imaging station, and return them to a stack, which is typically a further well plate storage cassette. Moreover, a biological sample container feeder/stacker, which may be well plate feeder/stacker, is preferably also provided to automate the replating operations. The biological sample container feeder/stacker is operable to supply each of a plurality of biological sample containers from a further feed to a replating station and return them to a further stack. For some applications, multiple feeder/stackers for replating may be provided.

For some classes of application, full automation may not be required. In particular, automated well plate feeding and stacking may not be needed. For example, when the source well plate has a large number of wells (96 or more) dispensing into 4 destination well plates also with a large number of wells, the transfer operation from that single well plate may take several hours including incubation periods. For such applications, manual placement of well plates on the bed of the robot may be adequate.

To perform the replating there is provided a cell picking head provided having at least one hollow pin for aspirating animal cells, and a head positioning system operable to move the cell picking head to allow replating of animal cells from a target well plate to a destination sample container. The invention thus envisages use of a robot equipped with an animal cell picking head comprising at least one hollow pin for aspirating animal cells and replating them by moving them from a target well plate, or other biological sample container to a destination biological sample container under the control of a head positioning system, the use comprising: providing a well plate in which animal cells are being cultured; arranging the well plate at an imaging station and detecting the degree of confluence in each well by repeatedly: (i) illuminating a selected well from below at an oblique angle; (ii) acquiring an image of the illuminated well; and (iii) processing the image of the well to determine the degree of confluence; and dependent on the degree of confluence, either replating the animal cells out of the well plate, or leaving them to continue to culture.

In an embodiment, the robot is equipped with at least one automated well plate supply mechanism, and the use comprises: providing a plurality of well plates in which animal cells are being cultured; supplying each well plate in turn to an imaging station, and at the imaging station detecting the degree of confluence in each well by repeatedly: (i) illuminating a selected well from below at an oblique angle; (ii) acquiring an image of the illuminated well; and (iii) processing the image of the well to determine the degree of confluence; and dependent on the degree of confluence, either replating the animal cells out of the well plate in which they are located, or leaving them to continue to culture.

The replating may be decided upon on individually for each well based on the degree of confluence of the well exceeding a confluence threshold. Alternatively, the replating may be decided upon on a well plate specific basis, in which replating is performed if a threshold number of wells exceed a confluence threshold, which may be 1, or a higher number.

### BRIEF DESCRIPTION OF THE DRAWINGS

For a better understanding of the invention and to show how the same may be carried out reference is now made by way of example to the accompanying drawings in which:
Figure 1 is a perspective view of an apparatus embodying the invention;
Figure 2 is a schematic sectional side view showing principles of the design of an optical sub-assembly for collecting well plate images for confluence detection;
Figure 3 is a schematic plan view of the optical design of Figure 2;
Figure 4 is a flow diagram showing the main steps of a process of imaging the wells of a well plate using the apparatus of Figure 1;
Figures 5A, 5B and 5C are perspective and orthogonal side views of the optics sub-assembly arranged below the main bed of the apparatus of Figure 1;
Figure 6 is a perspective view of the cell picking head of the apparatus;
Figure 7 is a perspective view of one pin of the head with associated agitation motor;
Figure 8 is a schematic section of the pin and motor of Figure 7 in use;
Figure 9 shows schematically the main steps in picking adherent animal cells from a well plate and moving to a further well plate with larger wells;
Figure 10 is a schematic drawing of the fluidics elements of the apparatus;
Figure 11 is a block schematic diagram showing the control system of the apparatus;
Figure 12 is a flow diagram showing an example process carried out by the apparatus; and
Figure 13 is a flow diagram of the cell picking part of the process of Figure 12.

### DETAILED DESCRIPTION

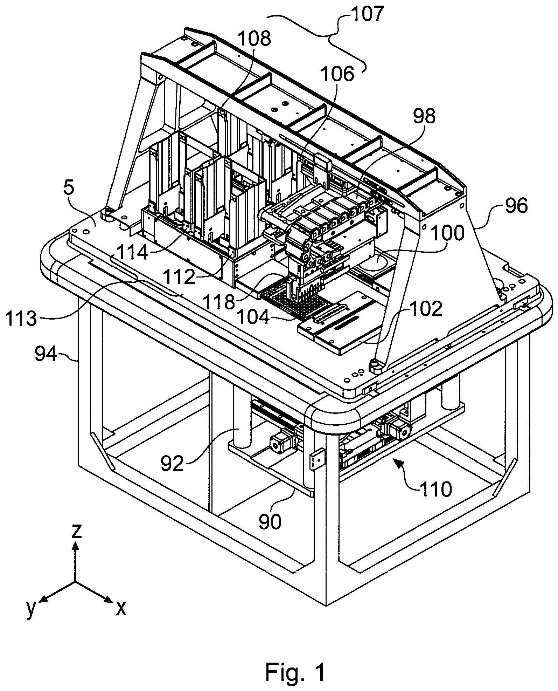

Figure 1 is a perspective view of an apparatus embodying the invention.

The apparatus may be considered to be a picking robot with integrated confluence detection optics. The apparatus can be subdivided notionally into two half spaces existing above and below a main bed 5 which is supported by a frame 94.

Above the main bed 5, the apparatus appears as similar to a conventional picking robot. A cell picking head 118 is provided that comprises a plurality of hollow pins for aspirating animal cells. The cell picking head 118 is movable over the main bed 5 by a head positioning system made up of x- y- and z-linear positioners 98 connected in series and suspended from a gantry 96. A wash/dry station 102 is also provided on the main bed 5 for cleansing the pins. The whole upper half space of the apparatus will typically be enclosed in a housing (not shown) including a hinged door extending over one side and part of the top of the apparatus.

Below the main bed 5, an optics sub-assembly 110 is provided to accommodate confluence detecting optics system which is mounted on a tray 90 suspended from the main bed 5 by pillars 92. The under-slung optics system is arranged to view well plates placed on the imaging station 100.

The main bed 5 is provided with two main working stations, namely an imaging station 100 and a replating station 104, each of which is positioned at the end of a respective well plate feed lane. Each well plate feed lane has a well plate feeder/stacker. The well plate feeder/stacker 107 for the imaging station 100 has a well plate feed storage cassette 106 and well plate (re-)stack storage cassette 108. A stack of well plates are held in the feed storage cassette 106, fed in turn down the lane via a delidder (not shown) to the imaging station 100, returned back along the lane, relidded and passed into the rear storage cassette 108. A similar well plate feeder/stacker 113 is used for the other lane to supply well plates from the storage cassette 112 to the replating station 104 and back along the lane to the (re-)stack storage cassette 114.

The well plate feeder/stacker mechanisms including delidding are described fully in EP-A-1 293 783 [2], the contents of which are incorporated herein by reference.

The cell picking head 118 can thus be moved from the imaging station to the replating station to allow replating of animal cells from a target well plate to a destination well plate. In the illustrated embodiment, there is only one destination lane. However, it may be desirable in some cases to have 2, 3 or 4 destination lanes. This may be useful when it is desired to split the animal cells from a given target well into multiple destination wells. The feeder/stacker mechanism is fully modular, so the number of well plate feed lanes can be increased without difficulty.

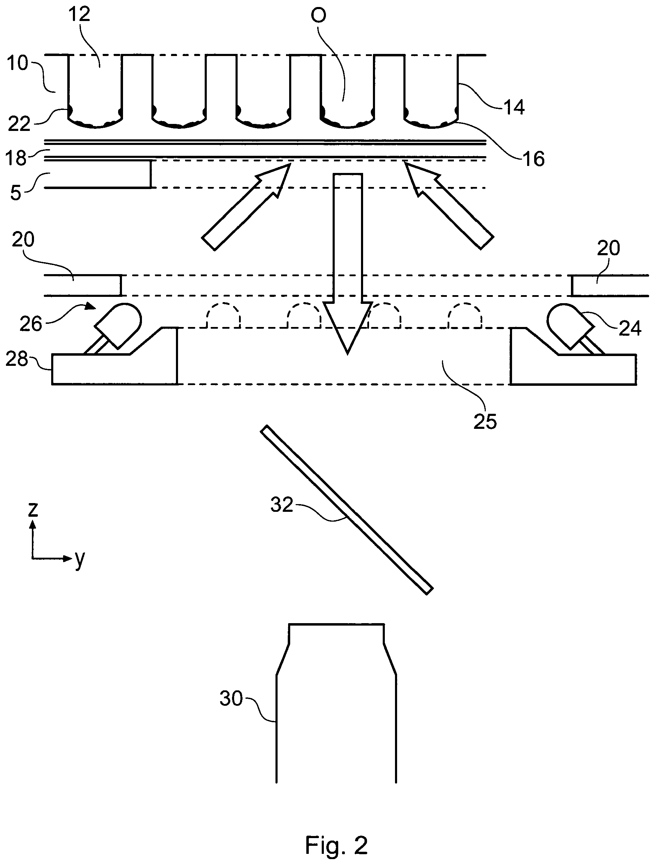

Figure 2 is a schematic sectional side view showing principles of the design of the optical sub-assembly 110 for collecting well plate images for confluence detection. Part of a well plate 10 showing 5 wells is also shown. Adherent colonies 22 have been cultured in the wells also as shown, the colonies forming around the base 16 and lower sidewalls 14 of the wells 12. The imaging station is formed in an aperture in the main bed 5 covered by a sheet of optically transparent material, typically glass, that forms a light table 18. For imaging, a well plate 10 is arranged on the light table 18 as shown, having been deposited there by the well plate feeder/stacker. The apparatus is designed to image one well at a time. To image a specific well 12 of a well plate, the optical sub-assembly 110 is aligned relative to the well 12.

The optical sub-assembly 110 comprises an illumination part and a collection part. The illumination part is formed of a plurality of white light emitting diodes (LEDs) 24 arranged to form an LED ring 26 located in a collar 28 with a central aperture 25 with the optical axes of the LEDs lying on the surface of a common cone, the point of which is coincident and labeled as the object position O in the figure. An apertured top plate 20 lying above the LED ring 26 is also illustrated. This is a structural component and has no significance for the optical design. The collection part of the optical sub-assembly is made up of a zoom lens 30 with autofocus. The optical axis is vertical and coincident with the object position O. A semi-silvered mirror 32 is also illustrated. This is for integrating a second illumination source (not shown) from the side onto the sample in order to perform fluorescence measurements.

The well to be imaged is thus aligned laterally with the optical axis of the collection optics and laterally and vertically with the center point of illumination, whereby the center point of illumination is around the base of the well or slightly higher as illustrated. The LEDs 24 thus illuminate a well 12 arranged in the object position O at an oblique angle from below so that an image of the well 12 is taken in a dark field configuration where light from the LEDs, if not scattered, does not contribute to the well image gathered by the collection lens 30.

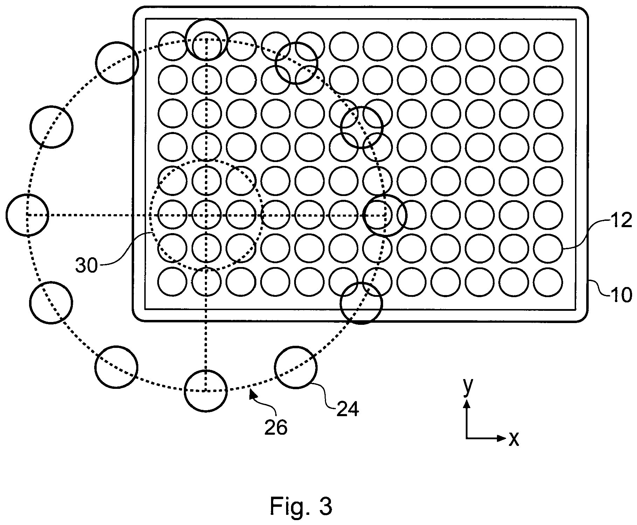

Figure 3 is a schematic plan view of selected parts of the optical system shown in Figure 2. The well plate 10 is a 96 well version and is shown aligned with the optical sub-assembly 110 so that a well 12 three rows up (row m=3) and two columns along (column n=2) is targeted, as illustrated by the objective lens 30 and LED ring 26 of LEDs 24. The optical sub-assembly is arranged on x- and y-positioners so that the collection lens 30 and illumination ring 26 can be moved together to image any one of the wells 12. Typically, the wells will be imaged in sequence row-wise and column-wise with a rastering process. This is achieved by moving the optical sub-assembly while the well plate remains static which is preferable so that liquid in the wells is not shaken by moving the well plate between imaging each well which might have an adverse influence on the imaging.

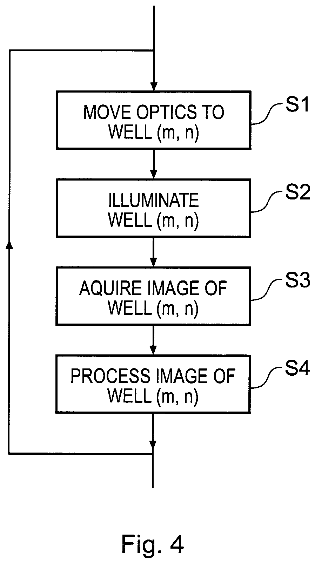

Figure 4 is a flow diagram showing the main steps of a process of imaging the wells of a well plate using the apparatus of Figure 1. In Step S1, the optical sub-assembly 110 is moved into alignment with well (m,n). In Step S2, the LED ring 26 is switched on to illuminate the well from below at an oblique angle defined by the LED arrangement. In Step S3, an image of the well (m,n) is collected through the lens 30, which leads to a CCD camera (not shown). In Step S4, the well image is processed by image processing software to determine the degree of confluence of the animal cells in the well.

To determine confluence, first the image is divided into background and foreground by extracting the background. This is done by applying a large Gaussian blur filter to the image, then subtracting this from the original image before adding the mean of the original image to each pixel. After this operation, pixels with intensities close to the mean of the resulting image are considered background, the remainder are considered foreground. The closeness to the mean is adjustable to accommodate variations in lighting etc. A segmented binary image is then generated by assigning foreground pixels to white and background pixels to black.

We envisage measuring the degree of confluence in one of two ways.

The first way involves counting the cells, and then assuming a value for cell area. This is usually reliable, since the variance in average cell area of a given cell type is usually small. The degree of confluence is then calculated to by the number of cells multiplied by the cell area divided by the available area of the well or other substrate, plate or dish.

The second way is to directly measure the aggregate area of all the cells by image processing of each individual cell to determine its boundary and thus area. The area of the cells can then be scaled up by a packing factor, e.g. assuming hexagonal close packing, before being divided by the available area of the well to arrive at a degree of confluence.

In the flow diagram, the image processing step, Step S4, is shown in the same loop as the image acquisition step, Step S3. It will be understood that these two steps need not be coupled in the same loop. For example the image processing can be done decoupled from the image acquisition, either one after the other, or in parallel.

The optics sub-assembly 110 is now described in more detail.

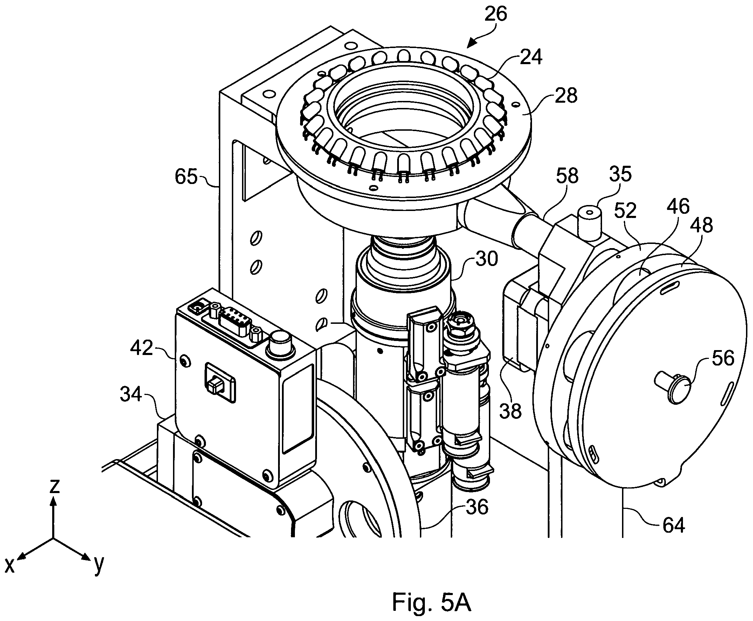

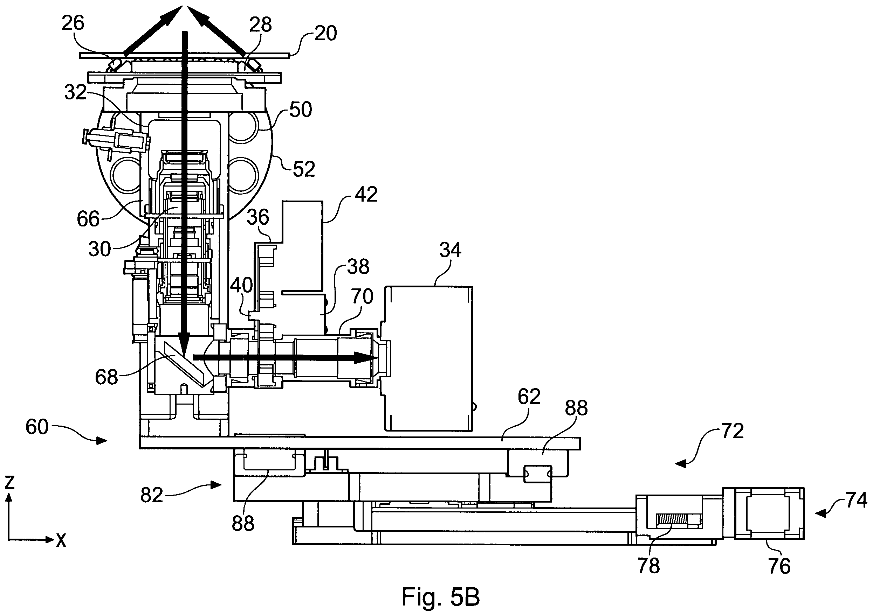

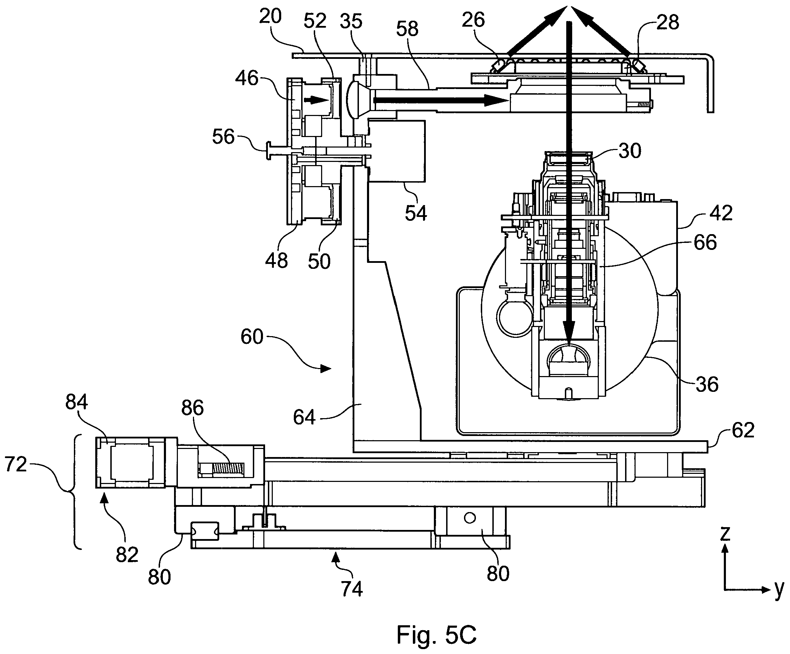

Figures 5A, 5B and 5C are perspective and orthogonal side views of the optics sub-assembly arranged below the main bed of the apparatus of Figure 1. These three figures are described together, rather than in turn, since they are different views of the same equipment, noting that not all features are visible or marked with reference numerals in each figure.

The previously described collar-mounted LED ring 24, 26, 28 is evident in all three figures. The LED collar 28 is cantilevered out on a side bracket from a vertical mounting plate 65 (Figure 5A) which is part of a frame 60. The vertical mounting plate 65 is upstanding from a base plate 62. The base plate 62 is also a platform for a further vertical mounting plate 64 for colored LEDs 44 (not shown) that are arranged in groups of different colors 46 on a wheel 48 which is a converted filter wheel with LED groups 46 arranged at each filter position. In front of each LED group 46 there is a bandpass or other suitable narrowband filter 50 (see Figures 5B & 5C) each arranged in the filter position of a further filter wheel 52 arranged coaxially and on the same motor spindle 56 as the filter wheel 48, the two wheels being driven in unison by a motor 54. Each bandpass filter 50 is selected to transmit a range of wavelengths matched to the emission wavelength band of the LED group 46 with which it is paired. Light from the uppermost LED group 46 is directed horizontally through a light pipe 58, which is not a waveguide, merely a shroud for preventing light spillage, onto the semi-silvered mirror 32 (see Figure 5B and also Figure 2) which serves as a beam splitter for directing a portion of the colored LED light through the LED collar's aperture 25 to the object position. Other forms of beam splitter could also be used, for example a cubic beam splitter. The beamsplitter is preferably removable, or movable away from the aperture 25 so that when lateral illumination from the colored LED groups is not needed, it can be taken out of the collection path so that it does not result in loss of collected signal. A mounting stub 35 is also evident in Figures 5A and 5C. This mounting stub 35 is for connecting the colored LED group features to the top plate 20 (removed in Figure 5A, but shown in Figures 5B and 5C and also Figure 2).

The collection lens 30 is held vertically in a mounting tube 66 (see Figures 5B & 5C) at the base of which is arranged a plane deflecting mirror 68 which redirects the collected light horizontally and supplies it along a light pipe 70 to a CCD camera 34. Part way along the light pipe 70 there is arranged a filter wheel 36 mounted on a spindle 40 and driven by a motor 38. Drive electronics for the filter wheel 36 are housed in a unit 42. Typically filters will be used in the collection optics to filter out excitation light from the colored LED groups 46 when spectroscopic measurements are being performed. Collection side filters may also be useful for filtering out fluorescence, e.g. to stop fluorescence from swamping out contrast of the cell periphery. This might be auto-fluorescence or fluorescence from a tag. For straightforward confluence detection using the white LEDs 24, no filter may be needed on the collection side.

The optical components are thus all mounted directly or indirectly on the base plate 62. The base plate 62 is carried by a linear positioner 82 which is in turn carried by a linear positioner 74 to provide xy-motion for the whole optical set-up, thereby providing collectively an optics positioning system 72. In the illustration, the x-positioner 74 is at the bottom with the y-positioner mounted on top of it. However, it will be appreciated this choice is arbitrary. It will also be appreciated that a parallel mechanism xy-positioner could be provided instead of two piggy-backed linear positioners. The x-positioner 74 comprises a motor 76, lead screw 78 and a pair of sets of guide bearings 80. The y-positioner 82 is the same, comprising a motor 84, lead screw 86 and a pair of sets of guide bearings 88.

As an alternative to having colored LED of different colors arranged in filter positions on a filter wheel as described above, it is possible to have concentric rings of different colors of LED in a single mounting. For example, the white light LED ring could be exchanged or supplemented with a number of LED rings of different colors. In principle an arbitrary arrangement of LEDs of different colors would provide the same functionality so long as LEDs of different colors could be driven independently, but would be a less elegant design. It would also be possible to use a single group of broadband LEDs in combination with filtering. However, this approach would tend to provide less illumination power than using different colors of LED. It will also be appreciated that other optical sources could be used including superfluorescent LEDs or diode lasers. Fixed wavelength or tunable diode lasers may be used.

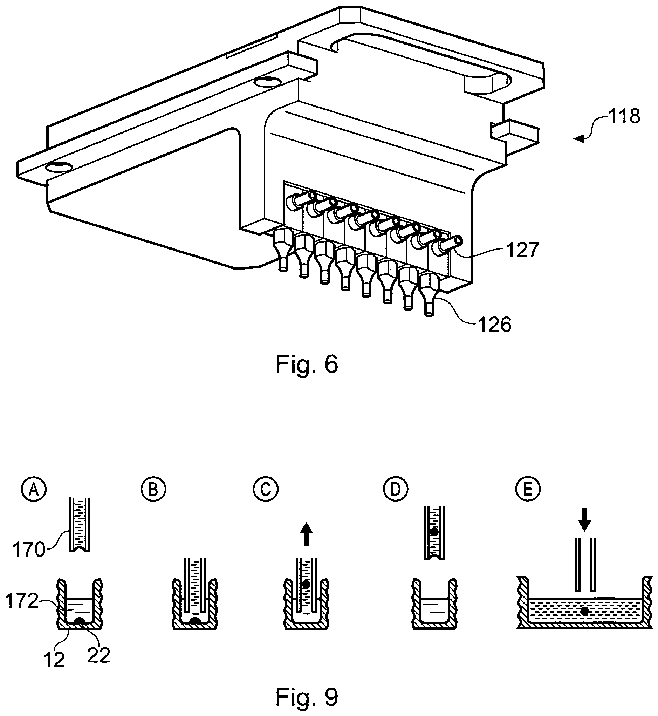

Figure 6 is a perspective view of the cell picking head 118 of the apparatus first shown in Figure 1. The end portions of the hollow pins 126 are visible protruding through the bottom of their housing parts. The hollow interior of the pins extends upwards and then through a right angle bend to emerge at stubs 127 for connecting flexible fluid lines (not shown in Figure 5). It is also noted that the head 118 with pins can be removed as a single piece and autoclaved for sterilization.

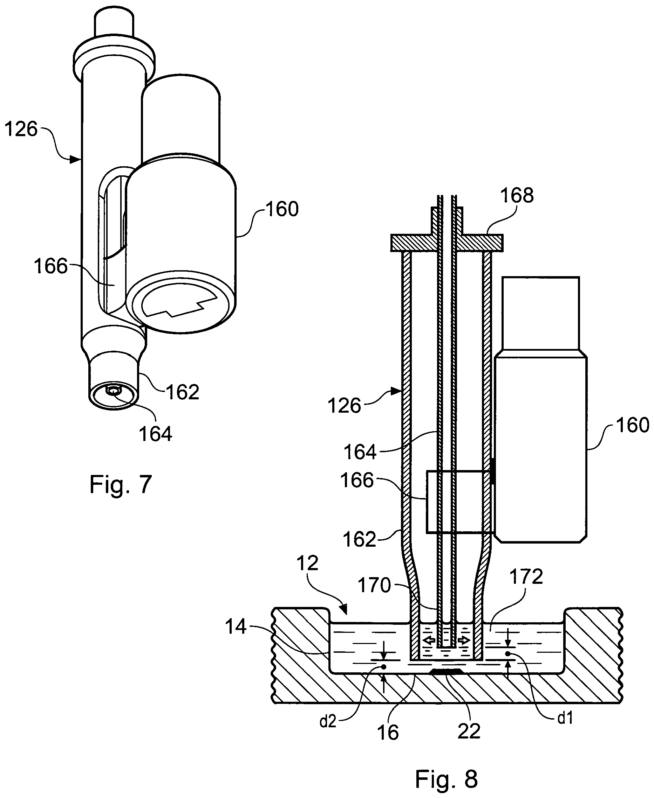

Figure 7 is a perspective view of one of the pins 126 in more detail. As well as the pin an electric motor 160 is also shown fitted alongside the pin 126. The hollow pin 126 comprises both outer and inner pins 162 and 164, with the inner pin 164 arranged coaxially inside the outer pin 162. Both pins are made of stainless steel. The inner pin 162 forms the end of the fluid path that includes the flexible tubing. The electric motor 160 is connected to the inner pin 164 by a connecting rod 166 that is driven by a crank mounted on the motor 160. The motor thus drives the inner pin 162 so that it describes a rotary motion, with the motion being accommodated by bending of the inner pin 164.

In the present embodiment, the inner pin 164 has an inside diameter of 0.7 mm an outside diameter of 1.07 mm. The outer pin 162 is 35 mm long and has a 5 mm outer diameter, tapering to 4.2 mm at its end, and a 3.2 mm inner diameter. These dimensions are suitable for picking cell colonies or other cell aggregates of average size circa 0.5 mm.

Figure 8 is a schematic section of the pin 126 and motor 160 showing the pin in use for picking adherent cells from a well 12. The inner and outer pins 164 and 162 can be seen, as well as the motor 160 and connecting rod 166. As is shown, the end of the inner pin 164 is recessed axially inside the end of the outer pin 162 by a distance 'd1'. In the present embodiment values of d1=0.25 - 0.5 mm have been used. Other values in the range 0.1 to 2 mm could be suitable, depending on the relevant parameters such as average cell colony or other cell aggregate size and liquid viscosity. The figure shows the pin 126 lowered in position for picking adherent cells 22. The pin 126 is lowered so that the distal end of the outer pin 162 is immersed in the liquid 172 and offset by a small amount 'd2' from the upper surface of the sample container base (substrate) 176 on which the cells are grown. In the present embodiment, d2 is varied between about zero (i.e. butting against the well plate base 16) and 0.5 mm, although larger offsets could be contemplated, for example up to 2 mm. Values of zero (i.e. butting), 0.1 mm or 0.2 mm are usual. Optionally, the pin 126 is specifically positionally aligned with the cells 22 as determined by the xy coordinates of the cells within the well, as determined by the image of the sample taken with the camera when determining confluence. Otherwise, the pin 126 is simply aligned centrally over the well.

In the position illustrated, the motor 160 is actuated to oscillate the end of the inner pin 164, thereby creating turbulence in the liquid 172 in which the cells are being cultured. An oscillation frequency of around 100 Hz has been successfully used. Other frequencies would probably also work. The forces induced by this motion have been found sufficient to detach the cells and allow aspiration of the detached cells into the hollow pin, which as mentioned above forms the end of a capillary 170. The inner pin 164 is constrained by a flange 168 which fits into the top of the outer pin 162 and has a central through hole through which the inner pin 164 passes in a push fit.

Another way of assisting detachment mechanically is by tapping, knocking or otherwise applying a mechanical shock to the well plate to dislodge cells, wherein this mechanical shock may be applied manually or through automation.

As an alternative to, or in combination with, mechanical detachment methods, adherent cells may be detached chemically, for example using buffers, salt solutions, detergents or biological materials, such as enzymes. Example media that can be placed in the wells to increase the efficiency by which cells can be dislodged are either an isotonic buffer containing different concentrations of divalent ions, or a buffer containing enzymes such as trypsin or proteases for releasing the cells from solid substrate. These media can be dispensed by a tube on the robotic head from the reservoir. After a period of incubation, normally between 5 and 20 minutes, a further medium may be added from another tube on the robotic head to stop the dissociation process. This isotonic medium may contain protein or divalent cations. The cell suspension can then be aspirated by a further tube on the robotic head and a measured aliquot of the cells dispensed into one or more wells in a destination well plate or multiple destination well plates. To assist incubation of an enzyme used to promote detachment, the well plate can beneficially be provided with a heated carrier element, such as a platen. For example, trypsin can be maintained at around 37 degrees Celsius to speed up its activity.

The above description has taken the example of an adherent cells. It will be understood that when cells are not adherent, a simplified form of the same process can be carried out with the steps associated with detaching an adherent cells being omitted. It will also be understood that some of the parts of the apparatus are redundant in the case that mechanical detachment of adherent cells is not needed and these parts could be omitted. For example, the outer pin could be omitted as well as the motor and associated drive parts.

Figure 9 is a series of schematic captions illustrating the main steps in picking an adherent animal cells from a well plate and moving to a further well plate with larger wells. In caption A, the pin 170 is being lowered over adherent target cells 22 immersed in liquid 172 after alignment of the pin in xy with the target well 12. (The target cells are shown in the illustration as a roughly hemispherical shape, but it will be understood that a typical adherent colony will be spread over the base of the well and be generally flat.) In caption B, the pin tip is immersed directly over the cells ready for carrying out the detachment process. Caption C shows the situation after the vibration-induced detachment in which aspiration is taking place. A column of liquid in which the cells are in suspension is drawn up into the pin by lowering of pressure in the pin. Caption D shows the situation after the pin has been raised out of the sample container. The cells are retained by a lower than atmospheric pressure being maintained in the pin. The head can then be moved over to a destination well plate with larger wells held at the replating station 104 (see Figure 1) for dispensing. Caption E shows the situation in which the end of the pin has been lowered into a well of the destination well plate, and the cells ejected from the pin by raising the pressure in the pin, thereby completing the replating operation.

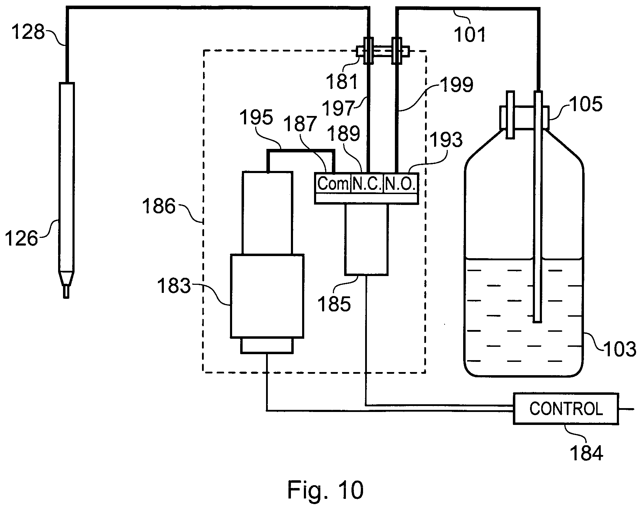

Figure 10 is a schematic drawing of the fluidics elements of the apparatus. One of the pins 126 is shown connected to its fluid line 128 made of flexible tubing which leads to a manifold 181 mounted on the housing of a fluidics unit 186. (The manifold 181 is used for receiving all the fluid lines 128, although only one is shown.) The manifold 181 also receives a further fluid line 101 from a liquid supply vessel 103 through a fluid line 101 which accesses the liquid in the supply vessel 103 through a sealed top flange 105. The liquid in the vessel 103 may be held under pressure. In the interior of the fluidics unit 186, the fluid supply is connected from the manifold 181 through a fluid line 199 to a normally open (N.O.) port 193 of a valve 185, and the other side of the fluid path leading to the pin 126 is connected from the manifold 181 through a fluid line 197 to a normally closed (N.C.) port 189 of the valve 185. The fluidics unit 186 also houses a pump 183, which is a reciprocating piston/cylinder pump which connects through a fluid line 195 to a common port (COM) 187 of the valve 185. The valve 185 and also the pump 183 are connected to a fluidics control unit 184 by electrical control lines. The fluidics control unit 184 is itself connected to and driven by a control computer (not shown in this figure).

In use, the valve 185 is controlled as follows. When the valve 185 is in its rest state with no inputs, the N.O. port 193 is open and the N.C. port 189 is closed. This connects the reciprocating pump 183 to the fluid vessel 103 so that it can draw liquid out of the reservoir by suitable downward motion of the pump piston in its cylinder. On the other hand, when the valve 185 is in its actuated state with an energizing input signal, the N.O. port 193 is closed and the N.C. port 189 is open. This connects the reciprocating pump 183 to the pin 126 allowing the liquid column in the fluid path formed by elements 126, 128, 197 and 195 to be moved in either direction by motion of the pump cylinder. This provides the fine control for the aspiration and expulsion of animal cells shown schematically in Figure 9 as described above. Raising of the piston also allows purging of the fluid path during cleaning. For cleaning, the fluid vessels can be swapped by hand. Alternatively, an automated switching between different fluid vessels can be provided using additional computer-controlled valves. On occasion, a pressurized gas canister could be used as a fluid vessel (e.g. for compressed gas cleaning), so the fluid vessels need not necessarily be liquid containing. It will be understood that the vessels 103 can be shared among multiple pins or separate vessels 103 can be provided for different pins, e.g. to supply different solutions to different pins. Individual pumps could also be shared between two or more valves to reduce cost.

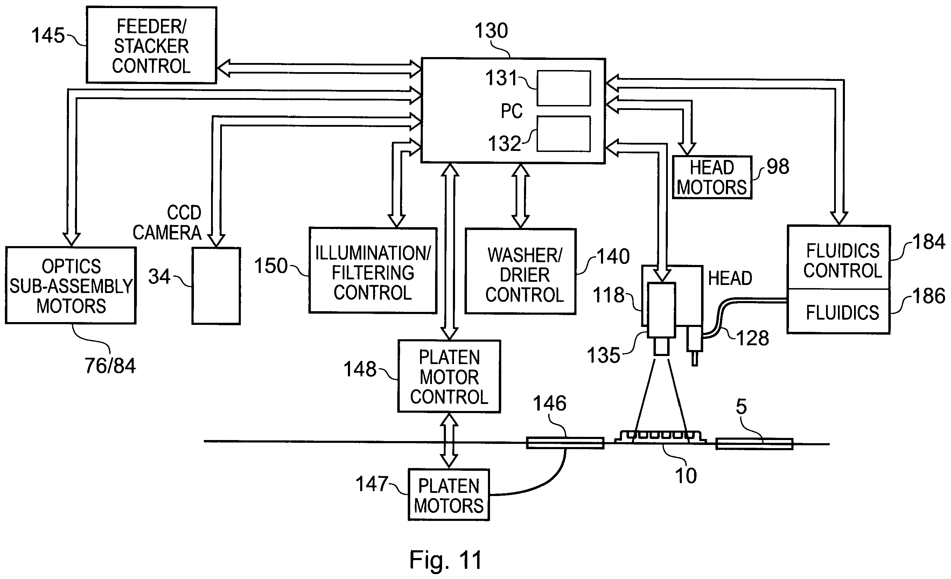

Figure 11 is a block schematic diagram showing the control system of the apparatus for coordinating the various components to perform the processes described above. A computer (PC 130) is used as the principal control component and is connected by electronic links using standard interfacing protocols to the various components that are part of the automated control system. The control is effected by control software 131 resident in the PC 130. Image processing software 132 is also resident in the PC 130 and linked to the control software 131. The image processing may be carried out in software, as just mentioned, hardware or firmware as desired. The CCD camera 34 is connected to the PC 130 for receiving digital images captured by the camera 34. An illumination and filter controller 150 is connected to the PC 130 for controlling the various under-bed optical sources and filter wheels of the optical sub-assembly 110. A washer/drier controller 140 is connected to the PC 130 and used to control the blower and the halogen lamps of the wash/dry station 102. The positioners 98 for moving the head 118 are connected to the PC 130. The PC 130 is also connected to the motors 76 and 84 of the x- and y-positioners of the under-bed optics sub-assembly 110. A head-mounted camera 135 is also provided for machine vision, such as bar-code detection on well plates, and is connected to the PC 130 for receiving digital images captured by the head-mounted camera 135. These are used for aligning the pins of the head with the various locations of interest such as the wash/dry station 102, well plates etc. The fluid lines 128 are connected to the fluidics unit 186 which is controlled by the fluidics control unit 184 connected to the PC 130. The fluidics control unit 184 is used to control the pressure in the fluid lines to allow aspiration, retention and expulsion of liquid from the sample. The fluidics control unit 184 also controls the wash cycle of the pins and fluid lines, whereby cleaning fluid from the baths is aspirated and expelled from the ends of the pins during the cleaning cycle. A feeder/stacker control unit 145 is also provided for the feeder/stacker units, including the well plate supply lanes, and is connected to the PC 130. Separate units 145 may be provided for each lane in view of the modular nature of the feeder/stacker assemblies. The figure also illustrates schematically an optional feature whereby a carrier in the form of a platen 146 is provided to carry one or more well plates 10 or other biological sample containers. The platen 146 is movable in the x- and y-directions by associated motors 147 and motor controller unit 148 which is connected to the PC 130, these elements collectively forming a positioning system for well plates or other containers arranged on the apparatus. The platen can then be moved in a controlled fashion to allow well-by-well iterative scanning by the optical system across all wells of a well plate. The platen may be provided with an integral heating element, so that well plates or other biological sample containers carried by the platen can be maintained at elevated temperatures, for example to promote enzymatic activity in the samples.

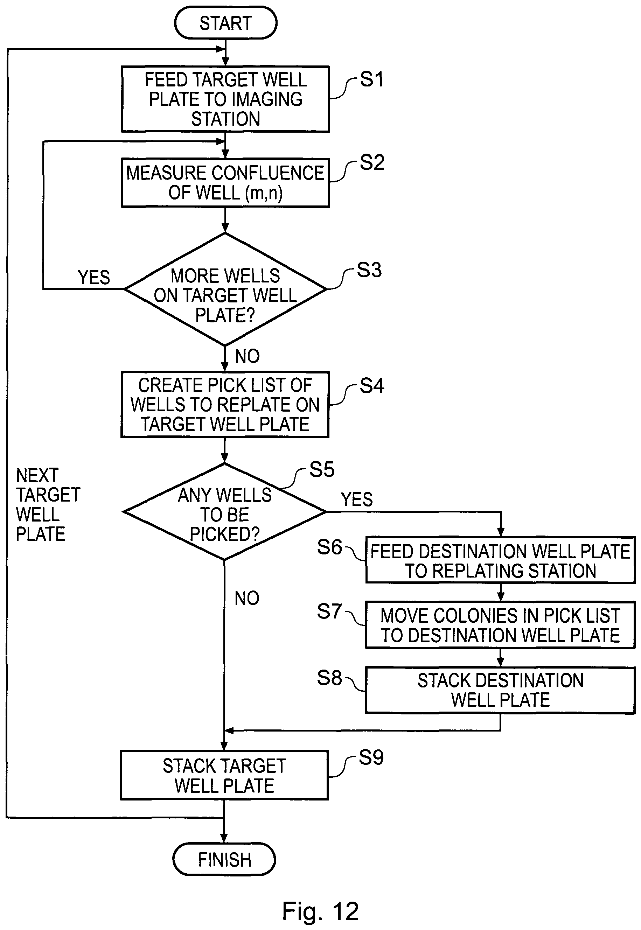

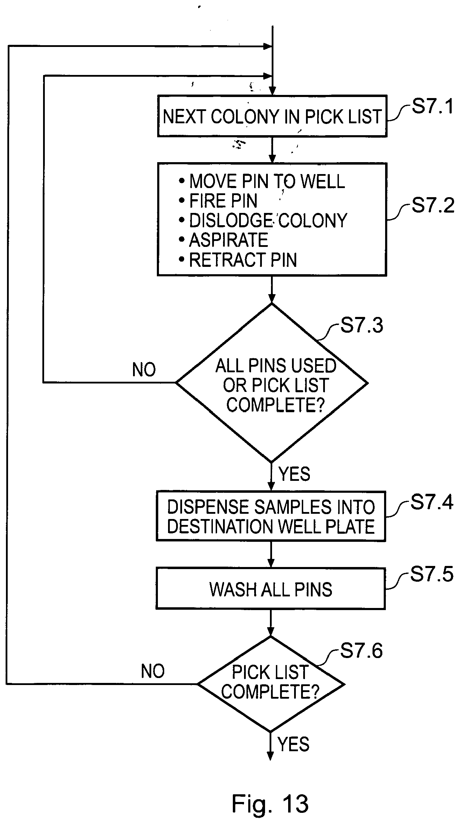

Figure 12 is a flow diagram showing an example process carried out by the apparatus. With reference to Figure 1, the process has the following manual pre-steps. A stack of well plates being cultured is loaded into a storage cassette and inserted in the robot in the feed slot of the imaging station lane, i.e. the position shown in Figure 1 by reference numeral 106. A suitable stack of empty well plates is loaded into the feed slot of the replating station lane, i.e. the position shown in Figure 1 by reference numeral 112. Moreover, two empty storage cassettes are inserted into the stack positions 108 and 114 to receive the processed well plates. The automated process which follows has the following steps:
- S1: feed a target well plate from the storage cassette 106 to the imaging station 100
- S2: measure the confluence of well (m,n) at the imaging station using the process described above with reference to Figure 4
- S3: if there are more wells to image return to Step S2, otherwise continue
- S4: based on the confluence measurements of the wells in the well plate compile a pick list of those wells which need to be replated
- S5: if the pick list is a null list then skip to Step S9, otherwise complete the replating by performing the following steps
- S6: feed a destination well plate from the storage cassette 112 to the replating station 104
- S7: move the cells across from the target well plate to the destination well plate following the pick list. The sub-steps of Step S7 are described below with reference to Figure 13.
- S8: stack the destination well plate by returning it along its lane to storage cassette 114. (Optionally, the destination well plate can be left at the replating station 104 if further colonies are to be loaded into it from further target well plates, and only stacked once it is full.)
- S9: stack the target well plate into storage cassette 108 so that the next target well plate can be delivered to the imaging station from storage cassette 106. If there are no more well plates in storage cassette 106 to image and detect confluence, then the process finishes.

Figure 13 is a flow diagram showing in more detail Step S7, the cell picking part of the process with reference to Figure 1 and also Figure 8.. Step S7 of the process has the following sub-steps:
S7.1 Select pick list element for picking
S7.2
   - Move an unused pin 126 to above the assigned xy coordinate of the well (m,n) to be picked
   - "Fire" (i.e. lower) pin to picking position with the end of the pin introduced into the medium over the target cells 22 by offset 'd2'
   - For adhered cells only - agitate pin end to dislodge target cells
   - Aspirate defined volume
   - Retract pin and retain sample while other pins are fired
S7.3 Unless all pins are used or all colonies in the pick list have been picked, repeat Step S7.2
S7.4 Move the head over to the destination well plate and align the pins with the wells - then dispense all the samples simultaneously into the destination well (microtiter) plate
S7.5 Clean all the pins using the washer and drier station 102
S7.6 Unless all colonies in the pick list have been picked move the head back to the imaging station to pick further colonies from the target well plate and return to Step S7.1.

This completes Step S7.

It will be understood that well plates with no wells identified as having reached threshold confluence will be returned to an incubator. This may be performed manually or in a semi- or fully automated way.

The process can be implemented as an expansion process to populate multiple well plates from a single well plate, optionally in multiple stages, such as 1 to 4, 4 to 16 etc. At each stage the well size may be increased, for example by using 96-well well plates in stage 1, 24-well well plates in stage 2, 6-well well plates in stage 3 and a single-well well plate or Petri dish (or other biological sample container) in stage 4. It can also be implemented as a consolidation process whereby positives (i.e. wells measured to be above threshold confluence) from a number of well plates are transferred into a sub-set of well plates, perhaps only a single well plate. It can also be implemented as a stratification process, whereby wells measured to be above threshold confluence are transferred to different target well plates depending on how rapidly they reached threshold, or depending on some other parameter. This can be used to separate fast, medium and slow growing cells or colonies of cells for example.

It will be appreciated that reference to well plates should be construed to include any receptacle in which the concept of confluence as described above is relevant.

### REFERENCES

1. De Blasio et al, Biotechniques 2004 April 36(4), pages 650ff "Combining optical and electrical impedance techniques for quantitative measurement of confluence in MDCK-I cell culture"
2. EP-A-1 293 783 (Genetix Limited)

## Claims

1. A process for determining animal cell confluence comprising:
arranging (S1) a biological sample container (10) in which animal cells (22) are being cultured in an object position (O) of an imaging station (100);
illuminating (S2) the object position (O) with an optical source (26) from below;
collecting (S3) light from the optical source (26) that has been scattered at the object position (O) to form an image of the biological sample container (10) arranged in the object position with a detector (34);
wherein, in said illuminating step, the object position (O) is illuminated at an oblique angle;
wherein said oblique angle at which the optical source (26) illuminates the object position (O) is between 10 to 50 degrees to the horizontal;
wherein, in said collecting step, the image is taken in a dark field configuration where light from the optical source, if not scattered, does not contribute to the image;
processing (S4) the image to determine the degree of confluence of the animal cells in the biological sample container (10), wherein the degree of confluence is the degree to which the animal cells have grown to fill the area of the biological sample container that is available for animal cell growth, and wherein said degree of confluence is determined:
by an automated cell count which is translated into an area by multiplication of the cell count by an area representing an average area for the cell type being cultured, or
by processing the image to: establish cell boundaries, compute the area of each cell from the cell boundary, and sum the cell areas.

2. The process of claim 1, wherein the oblique angle at which the optical source (26) illuminates the object position (O) is between 20 to 40 degrees to the horizontal.

3. The process of claim 1 or claim 2, wherein the biological sample container is a well plate (10) comprising a plurality of wells (12), and wherein the optical source (26) and the detector (34) are iteratively realigned relative to the well plate (10) so that images of a sequence of wells in the well plate are collected and processed, whereby the degree of confluence of the animal cells is determined in a plurality of wells across the well plate.

4. The process of any one of claims 1 to 3, wherein the optical source (26) comprises a plurality of directional light emitting units (24) arranged to emit beams having optical axes lying on the surface of a common cone, the point of which is coincident with the object position.

5. The process of any one of claims 1 to 3, wherein the optical source (26) comprises a plurality of directional light emitting units (24) arranged to emit beams having optical axes lying on the surface of at least two cones whose points are coincident with each other and the object position.

6. The process of claim 4 or 5, wherein the light emitting units are LEDs.

7. The process of claim 6, wherein the LEDs are white LEDs.

8. The process of any one of the preceding claims, wherein the image is collected from below the object position.

9. The process of any one of the preceding claims, wherein the optical source (26) is formed such that an open light path exists downwardly from the object position, and the light is collected via this open light path.

10. An apparatus for determining animal cell confluence comprising:
an imaging station (100) where a biological sample container (10) can be arranged in an object position (O);
an optical source (26) arranged to illuminate the object position (O) from below;
a detector (34) arranged to collect light from the optical source (26) that has been scattered at the object position (O) to form an image of a biological sample container (10) arranged in the object position;
and
an image processing unit (132) for processing images;
wherein the optical source is arranged to illuminate the object position at an oblique angle;
wherein said oblique angle at which the optical source (26) illuminates the object position (0) is between 10 to 50 degrees to the horizontal;
wherein the detector (34) is arranged to take the image in a dark field configuration where light from the optical source, if not scattered, does not contribute to the image;
wherein the image processing unit (132) is configured to determine the degree of confluence of animal cells (22) culturing in a biological sample container, wherein the degree of confluence is the degree to which the animal cells have grown to fill the area of the biological sample container that is available for animal cell growth, and
wherein the image processing unit is configured to determine said degree of confluence:
by an automated cell count which is translated into an area by multiplication of the cell count by an area representing an average area for the cell type being cultured, or
by processing the image to: establish cell boundaries, compute the area of each cell from the cell boundary, and sum the cell areas.

11. An apparatus according to claim 10, wherein the oblique angle at which the optical source (26) illuminates the object position (0) is between 20 to 40 degrees to the horizontal.

12. The apparatus of claim 10 or claim 11, further comprising:
an optics positioning system (72) for aligning the optical source (26) and the detector (34) relative to
the object position so that different parts of a biological sample container (10) arranged in the imaging station (100) can be moved into the object position.

13. The apparatus of any one of claims 10 to 12, further comprising:
a container positioning system (146, 147, 148) for aligning a biological sample container (10) arranged in the imaging station (100) relative to the object position so that different parts of a biological sample container arranged in the imaging station can be moved into the object position.

14. The apparatus of any one of claims 10 to 13, wherein the optical source (26) comprises a plurality of directional light emitting units (24) arranged to emit beams having optical axes lying on the surface of a common cone, the point of which is coincident with the object position.

15. The apparatus of claim any one of claims 10 to 13 , wherein the optical source (26) comprises a plurality of directional light emitting units (24) arranged to emit beams having optical axes lying on the surface of at least two cones whose points are coincident with each other and the object position.

16. The apparatus of claim 14 or 15, wherein the light emitting units are LEDs.

17. The apparatus of claim 16, wherein the LEDs are white LEDs.

18. The apparatus of any one of claims 10 to 17, wherein the detector (34) is positioned to collect light scattered downwardly from the object position.

19. The apparatus of claim 18, wherein the optical source (26) is formed such that an open light path exists downwardly from the object position, and the light is collected by the detector (34) via this open light path.

20. The apparatus of any one of claims 10 to 19, comprising a biological sample container feeder/stacker (107) operable to supply each of a plurality of biological sample containers from a feed (106) to the imaging station and return them to a stack (108).

21. The apparatus of claim 20, comprising a further biological sample container feeder/stacker (107) operable to supply each of a plurality of further biological sample containers (10) from a further feed to a replating station and return them to a further stack.

22. The apparatus of any one of claims 10 to 21, comprising a cell picking head (118) having at least one hollow pin (126) for aspirating animal cells, and a head positioning system (98) operable to move the cell picking head to allow replating of animal cells from a target biological sample container to a destination biological sample container.

23. The process of claim 3, or any one of claims 4 to 9 when dependent on claim 3, further comprising: providing a robot equipped with an animal cell picking head (118) comprising at least one hollow pin (126) for aspirating animal cells (22) and replating them by moving them from a target well plate to a destination biological sample container under the control of a head positioning system (98); providing a well plate (10) in which animal cells are being cultured; arranging the well plate at an imaging station (100) and detecting the degree of confluence in each well by repeatedly:
(i) illuminating a selected well from below at an oblique angle; (ii) acquiring an image of the illuminated well; and (iii) processing the image of the well to determine the degree of confluence; and dependent on the degree of confluence, either replating the animal cells out of the well plate, or leaving them to continue to culture.

24. The process of claim 23, wherein the replating is decided upon on individually for each well based on the degree of confluence of the well exceeding a confluence threshold.

25. The process of claim 23, wherein the replating is decided upon on a well plate specific basis, in which replating is performed if a threshold number of wells exceed a confluence threshold.

26. The process of claim 25, wherein the threshold number is 1.

27. The process of any one of claims 23 to 26, wherein the robot is further equipped with at least one automated well plate supply mechanism (106), which is used to supply each of a plurality of well plates in turn to the imaging station for confluence determination.

28. The process of any one of claims 23 to 27, wherein the animal cells are replated out of the well plate using a medium to assist dislodging the animal cells.

29. The process of claim 28, wherein the medium is a buffer containing divalent ions.

30. The process of claim 28, wherein the medium is a buffer containing enzymes.

31. The process of claim 30, wherein the medium is maintained at an elevated temperature to promote its activity in dislodging the animal cells.

32. The process of any one of claims 23 to 31, further comprising applying a mechanical shock to the well plate to assist dislodging of the animal cells.

## Patentansprüche

1. Prozess zum Bestimmen der Konfluenz von Tierzellen, umfassend:
Anordnen (S1) eines Behälters für biologische Proben (10), in dem Tierzellen (22) in einer Objektposition (O) einer Bildgebungsstation (100) kultiviert werden;
Beleuchten (S2) der Objektposition (O) mit einer optischen Quelle (26) von unten;
Erfassen (S3) des an der Objektposition (O) gestreuten Lichts von der optischen Quelle (26) mit einem Detektor (34), um ein Bild des in der Objektposition angeordneten Behälters für biologische Proben (10) zu erzeugen;
wobei im Beleuchtungsschritt die Objektposition (O) in einem schrägen Winkel beleuchtet wird;
wobei der schräge Winkel, in dem die optische Quelle (26) die Objektposition (O) beleuchtet, zwischen 10 und 50 Grad zur Horizontalen beträgt;
wobei im Erfassungsschritt das Bild in einer Dunkelfeldkonfiguration aufgenommen wird, in der Licht von der optischen Quelle, wenn es nicht gestreut ist, nicht zum Bild beiträgt;
Verarbeiten (S4) des Bildes, um den Grad der Konfluenz der Tierzellen im Behälter für biologische Proben (10) zu bestimmen,
wobei der Grad der Konfluenz der Grad ist, bis zu dem die Tierzellen gewachsen sind, um die für das Tierzellenwachstum verfügbare Fläche des Behälters für biologische Proben auszufüllen, und wobei der Grad der Konfluenz bestimmt wird:
durch eine automatische Zellzählung, die durch Multiplikation der Zellzählung mit einer Fläche, die eine Durchschnittsfläche für den kultivierten Zelltyp darstellt, in eine Fläche übersetzt wird, oder
durch Verarbeiten des Bildes zum: Feststellen von Zellgrenzen, Berechnen der Fläche jeder Zelle aus der Zellgrenze und Summieren der Zellflächen.

2. Prozess nach Anspruch 1, wobei der schräge Winkel, in dem die optische Quelle (26) die Objektposition (O) beleuchtet, zwischen 20 und 40 Grad zur Horizontalen beträgt.

3. Prozess nach Anspruch 1 oder Anspruch 2, wobei der Behälter für biologische Proben eine Mikrotiterplatte (10) ist, umfassend eine Vielzahl von Kavitäten (12), und wobei die optische Quelle (26) und der Detektor (34) bezüglich der Mikrotiterplatte (10) iterativ neu ausgerichtet werden, so dass Bilder einer Folge von Kavitäten in der Mikrotiterplatte erfasst und verarbeitet werden, wobei der Grad der Konfluenz der Tierzellen in einer Vielzahl von Kavitäten über die Mikrotiterplatte bestimmt wird.

4. Prozess nach einem der Ansprüche 1 bis 3, wobei die optische Quelle (26) eine Vielzahl gerichteter Leuchteinheiten (24) umfasst, die angeordnet sind, um Strahlen zu emittieren, deren optische Achsen auf der Oberfläche eines gemeinsamen Kegels liegen, dessen Spitze mit der Objektposition zusammenfällt.

5. Prozess nach einem der Ansprüche 1 bis 3, wobei die optische Quelle (26) eine Vielzahl gerichteter Leuchteinheiten (24) umfasst, die angeordnet sind, um Strahlen zu emittieren, deren optische Achsen auf der Oberfläche von mindestens zwei Kegeln liegen, deren Spitzen miteinander und mit der Objektposition zusammenfallen.

6. Prozess nach Anspruch 4 oder 5, wobei die Leuchteinheiten LEDs sind.

7. Prozess nach Anspruch 6, wobei die LEDs weiße LEDs sind.

8. Prozess nach einem der vorhergehenden Ansprüche, wobei das Bild von unterhalb der Objektposition erfasst wird.

9. Prozess nach einem der vorhergehenden Ansprüche, wobei die optische Quelle (26) so gebildet ist, dass ein offener Lichtweg von der Objektposition nach unten vorhanden ist und das Licht über diesen offenen Lichtweg erfasst wird.

10. Vorrichtung zum Bestimmen der Konfluenz von Tierzellen, umfassend:
eine Bildgebungsstation (100), bei der ein Behälter für biologische Proben (10) in einer Objektposition (O) angeordnet werden kann;
eine optische Quelle (26), die eingerichtet ist, um die Objektposition (O) von unten zu beleuchten;
einen Detektor (34), der angeordnet ist, um an der Objektposition (O) gestreutes Licht von der optischen Quelle (26) zu erfassen, um ein Bild eines in der Objektposition angeordneten Behälters für biologische Proben (10) zu erzeugen;
und
eine Bildverarbeitungseinheit (132) zum Verarbeiten von Bildern;
wobei die optische Quelle angeordnet ist, um die Objektposition in einem schrägen Winkel zu beleuchten;
wobei der schräge Winkel, in dem die optische Quelle (26) die Objektposition (O) beleuchtet, zwischen 10 und 50 Grad zur Horizontalen beträgt;
wobei der Detektor (34) angeordnet ist, um das Bild in einer Dunkelfeldkonfiguration aufzunehmen, in der Licht von der optischen Quelle, wenn es nicht gestreut ist, nicht zum Bild beiträgt;
wobei die Bildverarbeitungseinheit (132) dafür konfiguriert ist, den Grad der Konfluenz von in einem Behälter für biologische Proben kultivierten Tierzellen (22) zu bestimmen, wobei der Grad der Konfluenz der Grad ist, bis zu dem die Tierzellen gewachsen sind, um die für das Tierzellenwachstum verfügbare Fläche des Behälters für biologische Proben auszufüllen, und
wobei die Bildverarbeitungseinheit dafür konfiguriert ist, den Grad der Konfluenz zu bestimmen:
durch eine automatische Zellzählung, die durch Multiplikation der Zellzählung mit einer Fläche, die eine Durchschnittsfläche für den kultivierten Zelltyp darstellt, in eine Fläche übersetzt wird, oder
durch Verarbeiten des Bildes zum: Feststellen von Zellgrenzen, Berechnen der Fläche jeder Zelle aus der Zellgrenze und Summieren der Zellflächen.

11. Vorrichtung nach Anspruch 10, wobei der schräge Winkel, in dem die optische Quelle (26) die Objektposition (O) beleuchtet, zwischen 20 und 40 Grad zur Horizontalen beträgt.

12. Vorrichtung nach Anspruch 10 oder Anspruch 11, ferner umfassend:
ein Optikpositionierungssystem (72) zum Ausrichten der optischen Quelle (26) und des Detektors (34) bezüglich der Optikposition, so dass verschiedene Teile eines Behälters für biologische Proben (10), der in der Bildgebungsstation (100) angeordnet ist, in die Objektposition bewegt werden können.

13. Vorrichtung nach einem der Ansprüche 10 bis 12, ferner umfassend:
ein Behälterpositionierungssystem (146, 147, 148) zum Ausrichten eines Behälters für biologische Proben (10), der in der Bildgebungsstation (100) angeordnet ist, bezüglich der Objektposition, so dass verschiedene Teile eines Behälters für biologische Proben, der in der Bildgebungsstation angeordnet ist, in die Objektposition bewegt werden können.

14. Vorrichtung nach einem der Ansprüche 10 bis 13, wobei die optische Quelle (26) eine Vielzahl gerichteter Leuchteinheiten (24) umfasst, die angeordnet sind, um Strahlen zu emittieren, deren optische Achsen auf der Oberfläche eines gemeinsamen Kegels liegen, dessen Spitze mit der Objektposition zusammenfällt.

15. Vorrichtung nach einem der Ansprüche 10 bis 13, wobei die optische Quelle (26) eine Vielzahl gerichteter Leuchteinheiten (24) umfasst, die angeordnet sind, um Strahlen zu emittieren, deren optische Achsen auf der Oberfläche von mindestens zwei Kegeln liegen, deren Spitzen miteinander und mit der Objektposition zusammenfallen.

16. Vorrichtung nach Anspruch 14 oder 15, wobei die Leuchteinheiten LEDs sind.

17. Vorrichtung nach Anspruch 16, wobei die LEDs weiße LEDs sind.

18. Vorrichtung nach einem der Ansprüche 10 bis 17, wobei der Detektor (34) positioniert ist, um Licht zu erfassen, das von der Objektposition nach unten gestreut ist.

19. Vorrichtung nach Anspruch 18, wobei die optische Quelle (26) so gebildet ist, dass ein offener Lichtweg von der Objektposition nach unten vorhanden ist und das Licht über diesen offenen Lichtweg vom Detektor (34) erfasst wird.

20. Vorrichtung nach einem der Ansprüche 10 bis 19, umfassend eine Zuführ-/Stapeleinheit (107) für Behälter für biologische Proben, die zum Zuführen jedes einer Vielzahl von Behältern für biologische Proben von einer Zuführung (106) zur Bildgebungsstation und Rückführen derselben zu einem Stapel (108) betreibbar ist.

21. Vorrichtung nach Anspruch 20, umfassend eine weitere Zuführ-/Stapeleinheit (107) für Behälter für biologische Proben, die zum Zuführen jedes einer Vielzahl weiterer Behälter für biologische Proben (10) von einer weiteren Zuführung zu einer Umplattierungsstation und Rückführen derselben zu einem weiteren Stapel betreibbar ist.

22. Vorrichtung nach einem der Ansprüche 10 bis 21, umfassend einen Zellaufnahmekopf (118) mit mindestens einer Hohlnadel (126) zum Ansaugen von Tierzellen und einem Kopfpositioniersystem (98), das zum Verschieben des Zellaufnahmekopfes betreibbar ist, um ein Umplattieren von Tierzellen von einem Zielbehälter für biologische Proben in einen Bestimmungsbehälter für biologische Proben zu ermöglichen.

23. Prozess nach Anspruch 3 oder einem der Ansprüche 4 bis 9, wenn von Anspruch 3 abhängig, ferner umfassend: Bereitstellen eines Roboters, ausgestattet mit einem Tierzellen-Aufnahmekopf (118), umfassend mindestens eine Hohlnadel (126) zum Ansaugen von Tierzellen (22) und Umplattieren derselben, indem sie von der Ziel-Mikrotiterplatte in einen Bestimmungsbehälter für biologische Proben unter der Steuerung eines Kopfpositioniersystems (98) verschoben werden; bereitstellend eine Mikrotiterplatte (10), in der Tierzellen kultiviert werden; Anordnen der Mikrotiterplatte an einer Bildgebungsstation (100) und Erkennen des Grades der Konfluenz in jeder Kavität durch wiederholtes:
(i) Beleuchten einer ausgewählten Kavität von unten in einem schrägen Winkel; (ii) Erfassen eines Bildes von der beleuchteten Kavität; und (iii) Verarbeiten des Bildes der Kavität zum Bestimmen des Grades der Konfluenz; und abhängig vom Grad der Konfluenz entweder Umplattieren der Tierzellen aus der Mikrotiterplatte, oder Belassen derselben zum Weiterkultivieren.

24. Prozess nach Anspruch 23, wobei das Umplattieren individuell für jede Kavität einzeln entschieden wird, basierend auf dem Grad der Konfluenz in der Kavität, die einen Konfluenzschwellenwert überschreitet.

25. Prozess nach Anspruch 23, wobei das Umplattieren auf einer mikrotiterplattenspezifischen Basis entschieden wird, bei der ein Umplattieren durchgeführt wird, wenn eine Schwellenzahl von Kavitäten einen Konfluenzschwellenwert überschreitet.

26. Prozess nach Anspruch 25, wobei die Schwellenzahl 1 beträgt.

27. Prozess nach einem der Ansprüche 23 bis 26, wobei der Roboter ferner mit mindestens einem automatisierten Mikrotiterplatten-Zuführmechanismus (106) ausgestattet ist, der dazu dient, jede einer Vielzahl von Mikrotiterplatten der Reihe nach zur Bestimmung der Konfluenz der Bildgebungsstation zuzuführen.

28. Prozess nach einem der Ansprüche 23 bis 27, wobei die Tierzellen aus der Mikrotiterplatte umplattiert werden, indem ein Medium verwendet wird, das das Ablösen der Tierzellen unterstützt.

29. Prozess nach Anspruch 28, wobei das Medium ein Puffer ist, der zweiwertige Ionen enthält.

30. Prozess nach Anspruch 28, wobei das Medium ein Puffer ist, der Enzyme enthält.

31. Prozess nach Anspruch 30, wobei das Medium auf einer erhöhten Temperatur gehalten wird, um seine Aktivität beim Ablösen der Tierzellen zu fördern.

32. Prozess nach einem der Ansprüche 23 bis 31, ferner das Aufbringen eines mechanischen Stoßes auf die Mikrotiterplatte umfassend, um das Ablösen der Tierzellen zu unterstützen.

## Revendications

1. Procédé pour déterminer une confluence de cellules animales consistant à :
disposer (S1) un récipient pour échantillons biologiques (10) dans lequel des cellules animales (22) sont cultivées, dans une position d'objet (O) d'un poste d'imagerie (100) ;
éclairer (S2) la position d'objet (O) avec une source optique (26) par le bas ;
collecter (S3) la lumière provenant de la source optique (26) qui a été dispersée au niveau de la position d'objet (O) afin de former une image du récipient pour échantillons biologiques (10) disposé à la position d'objet avec un détecteur (34) ;
dans lequel, au cours de ladite étape d'éclairage, la position d'objet (O) est éclairée selon un angle oblique ;
dans lequel ledit angle oblique selon lequel la source optique (26) éclaire la position d'objet (O), varie entre 10 et 50 degrés par rapport à l'horizontale ;
dans lequel, au cours de ladite étape de collecte, l'image est prise dans une configuration de fond noir dans laquelle la lumière provenant de la source optique, si elle n'est pas dispersée, ne contribue pas à l'image ;
traiter (S4) l'image pour déterminer le degré de confluence des cellules animales dans le récipient pour échantillons biologiques (10),
dans lequel le degré de confluence est le degré selon lequel les cellules animales ont crû pour remplir la surface du récipient pour échantillons biologiques qui est disponible pour une croissance de cellules animales, et dans lequel ledit degré de confluence est déterminé :
par une numération cellulaire automatisée qui est traduite en surface par multiplication de la numération cellulaire par une surface représentant une surface moyenne pour le type de cellule qui est cultivé, ou
par traitement de l'image pour : établir des limites de cellule, calculer la surface de chaque cellule à partir de la limite de cellule et sommer les surfaces de cellule.

2. Procédé selon la revendication 1, dans lequel l'angle oblique selon lequel la source optique (26) éclaire la position d'objet (O) est compris entre 20 et 40 degrés par rapport à l'horizontale.

3. Procédé selon la revendication 1 ou 2, dans lequel le récipient pour échantillons biologiques est une plaque à puits (10) comprenant une pluralité de puits (12) et dans lequel la source optique (26) et le détecteur (34) sont réalignés de manière itérative par rapport à la plaque à puits (10) de telle sorte que des images d'une séquence de puits de la plaque à puits soient collectées et traitées de sorte que le degré de confluence des cellules animales soit déterminé dans une pluralité de puits d'un côté à l'autre de la plaque à puits.

4. Procédé selon l'une quelconque des revendications 1 à 3, dans lequel la source optique (26) comprend une pluralité d'unités d'émission de lumière directionnelle (24) conçues pour émettre des rayons ayant des axes optiques se trouvant sur la surface d'un cône commun dont la pointe coïncide avec la position d'objet.

5. Procédé selon l'une quelconque des revendications 1 à 3, dans lequel la source optique (26) comprend une pluralité d'unités d'émission de lumière directionnelle (24) conçues pour émettre des rayons ayant des axes optiques se trouvant sur la surface d'au moins deux cônes dont les pointes coïncident les unes avec les autres et avec la position d'objet.

6. Procédé selon la revendication 4 ou 5, dans lequel les unités d'émission de lumière sont des diodes électroluminescentes (DEL).

7. Procédé selon la revendication 6, dans lequel les DEL sont des DEL blanches.

8. Procédé selon l'une quelconque des revendications précédentes, dans lequel l'image est collectée par le bas de la position d'objet.

9. Procédé selon l'une quelconque des revendications précédentes, dans lequel la source optique (26) est formée de telle sorte qu'un trajet de lumière ouvert existe vers le bas depuis la position d'objet et que la lumière soit collectée par le biais de ce trajet de lumière ouvert.

10. Appareil pour déterminer une confluence de cellules animales comprenant :
un poste d'imagerie (100) où un récipient pour échantillons biologiques (10) peut être disposé dans une position d'objet (O) ;
une source optique (26) conçue pour éclairer la position d'objet (O) par le bas ;
un détecteur (34) conçu pour collecter la lumière provenant de la source optique (26) qui a été dispersée au niveau de la position d'objet (O) afin de former une image du récipient pour échantillons biologiques (10) disposé à la position d'objet ;
et
une unité de traitement d'image (132) pour traiter des images ;
dans lequel la source optique est conçue pour éclairer la position d'objet selon un angle oblique ;
dans lequel ledit angle oblique selon lequel la source optique (26) éclaire la position d'objet (O), varie entre 10 et 50 degrés par rapport à l'horizontale ;
dans lequel le détecteur (34) est conçu pour prendre l'image dans une configuration de fond noir dans laquelle la lumière provenant de la source optique, si elle n'est pas dispersée, ne contribue pas à l'image ;
dans lequel l'unité de traitement d'image (132) est configurée pour déterminer le degré de confluence des cellules animales (22) cultivées dans un récipient pour échantillons biologiques, dans lequel le degré de confluence est le degré selon lequel les cellules animales ont crû pour remplir la surface du récipient pour échantillons biologiques qui est disponible pour une croissance de cellules animales, et
dans lequel l'unité de traitement d'image est configurée pour déterminer ledit degré de confluence :
par une numération cellulaire automatisée qui est traduite en surface par multiplication de la numération cellulaire par une surface représentant une surface moyenne pour le type de cellule qui est cultivé, ou
par traitement de l'image pour : établir des limites de cellule, calculer la surface de chaque cellule à partir de la limite de cellule et sommer les surfaces de cellule.

11. Appareil selon la revendication 10, dans lequel l'angle oblique selon lequel la source optique (26) éclaire la position d'objet (0) est compris entre 20 et 40 degrés par rapport à l'horizontale.

12. Appareil selon la revendication 10 ou la revendication 11, comprenant en outre :
un système de positionnement d'optique (72) pour aligner la source optique (26) et le détecteur (34) par rapport à la position d'objet de telle sorte que différentes parties d'un récipient pour échantillons biologiques (10) disposé dans le poste d'imagerie (100) puissent être installées en position d'objet.

13. Appareil selon l'une quelconque des revendications 10 à 12, comprenant en outre :
un système de positionnement de récipient (146, 147, 148) pour aligner un récipient pour échantillons biologiques (10) disposé dans le poste d'imagerie (100) par rapport à la position d'objet de telle sorte que différentes parties d'un récipient pour échantillons biologiques disposé dans le poste d'imagerie puissent être installées en position d'objet.

14. Appareil selon l'une quelconque des revendications 10 à 13, dans lequel la source optique (26) comprend une pluralité d'unités d'émission de lumière directionnelle (24) conçues pour émettre des rayons ayant des axes optiques se trouvant sur la surface d'un cône commun dont la pointe coïncide avec la position d'objet.

15. Appareil selon l'une quelconque des revendications 10 à 13, dans lequel la source optique (26) comprend une pluralité d'unités d'émission de lumière directionnelle (24) conçues pour émettre des rayons ayant des axes optiques se trouvant sur la surface d'au moins deux cônes dont les pointes coïncident les unes avec les autres et avec la position d'objet.

16. Appareil selon la revendication 14 ou 15, dans lequel les unités d'émission de lumière sont des DEL.

17. Appareil selon la revendication 16, dans lequel les DEL sont des DEL blanches.

18. Appareil selon l'une quelconque des revendications 10 à 17, dans lequel le détecteur (34) est positionné de sorte à collecter la lumière dispersée vers le bas depuis la position d'objet.

19. Appareil selon la revendication 18, dans lequel la source optique (26) est formée de telle sorte qu'un trajet de lumière ouvert existe vers le bas depuis la position d'objet et que la lumière soit collectée par le détecteur (34) par le biais de ce trajet de lumière ouvert.

20. Appareil selon l'une quelconque des revendications 10 à 19, comprenant un dispositif d'alimentation/d'empilage (107) de récipient pour échantillons biologiques destiné à fournir chaque récipient pour échantillons biologiques d'une pluralité de récipients pour échantillons biologiques depuis une alimentation (106) au poste d'imagerie et à les renvoyer à une pile (108).

21. Appareil selon la revendication 20, comprenant un autre dispositif d'alimentation/d'empilage (107) de récipient pour échantillons biologiques destiné à fournir chaque récipient pour échantillons biologiques d'une pluralité d'autres récipients pour échantillons biologiques (10) depuis une autre alimentation à un poste de réensemencement et à les renvoyer à une autre pile.

22. Appareil selon l'une quelconque des revendications 10 à 21, comprenant une tête de capture de cellules (118) ayant au moins une broche creuse (126) pour aspirer des cellules animales, et un système de positionnement de tête (98) destiné à déplacer la tête de capture de cellules pour permettre un réensemencement des cellules animales passant d'un récipient pour échantillons biologiques cible à un récipient pour échantillons biologiques de destination.

23. Procédé selon la revendication 3 ou selon l'une quelconque des revendications 4 à 9 lorsqu'elle dépend de la revendication 3, consistant en outre à : fournir un robot équipé d'une tête de capture de cellules animales (118) comprenant au moins une broche creuse (126) pour aspirer des cellules animales (22) et les réensemencer en les déplaçant depuis une plaque à puits cible jusqu'à un récipient pour échantillons biologiques de destination sous la commande d'un système de positionnement de tête (98) ; fournir une plaque à puits (10) dans laquelle des cellules animales sont cultivées ; disposer la plaque à puits au niveau d'un poste d'imagerie (100) et détecter le degré de confluence dans chaque puits en effectuant à plusieurs reprises les opérations suivantes : (i) éclairant un puits sélectionné par le bas selon un angle oblique ; (ii) acquérant une image du puits éclairé ; et (iii) traitant l'image du puits afin de déterminer le degré de confluence ; et, en fonction du degré de confluence, soit réensemencer les cellules animales en dehors de la plaque à puits, soit les laisser poursuivre leur culture.

24. Procédé selon la revendication 23, dans lequel le réensemencement est décidé de manière individuelle pour chaque puits en se basant sur le degré de confluence du puits dépassant un seuil de confluence.

25. Procédé selon la revendication 23, dans lequel le réensemencement est décidé sur une base spécifique à une plaque à puits selon laquelle un réensemencement est réalisé si un nombre seuil de puits dépasse un seuil de confluence.

26. Procédé selon la revendication 25, dans lequel le nombre seuil est 1.

27. Procédé selon l'une quelconque des revendications 23 à 26, dans lequel le robot est en outre équipé d'au moins un mécanisme automatisé d'alimentation en plaque à puits (106) qui est utilisé pour fournir l'une après l'autre chaque plaque à puits d'une pluralité de plaques à puits au poste d'imagerie pour réaliser une détermination de la confluence.

28. Procédé selon l'une quelconque des revendications 23 à 27, dans lequel les cellules animales sont réensemencées en dehors de la plaque à puits à l'aide d'un milieu pour aider au délogement des cellules animales.

29. Procédé selon la revendication 28, dans lequel le milieu est un tampon contenant des ions divalents.

30. Procédé selon la revendication 28, dans lequel le milieu est un tampon contenant des enzymes.

31. Procédé selon la revendication 30, dans lequel le milieu est maintenu à une température élevée de sorte à favoriser son activité lors du délogement des cellules animales.

32. Procédé selon l'une quelconque des revendications 23 à 31, consistant en outre à appliquer un choc mécanique à la plaque à puits pour aider au délogement des cellules animales.
